# EUROPEAN PATENT APPLICATION

(11) **EP 4 169 563 A1**
(43) Date of publication of application: **26.04.2023**
(21) Application number: 20941357.4
(22) Date of filing: 17.06.2020
(51) Int. Cl.: A61M 25/092, A61B 34/30, A61M 25/00

(54) **LONG MEDICAL DEVICE**

(71) Applicant: Asahi Intecc Co., Ltd., Seto-shi, Aichi 489-0071 (JP)
(72) Inventor: YOSHIMATSU, Daiki, Seto-shi, Aichi 489-0071 (JP); OSHIMA, Fumiyoshi, Seto-shi, Aichi 489-0071 (JP); NAMIMA, Satoshi, Seto-shi, Aichi 489-0071 (JP); KUBO, Yuta, Seto-shi, Aichi 489-0071 (JP); NAKAMIZO, Kohei, Tokyo 160-0023 (JP)
(74) Representative: Winter, Brandl - Partnerschaft mbB
(86) International application number: PCT/JP2020/023859
(87) International publication number: WO 2021/255877

(57) **Abstract**

Make a distal end portion to bend easily. A catheter device 1 is configured to include a main body portion 4 that is bendable and is provided on a distal end side, two systems of wire ropes 20A, 20B coupled to a distal end portion of the main body portion 4 with a space in a predetermined first direction, and an operating portion 6 that is operable by a user operation and is coupled to a proximal end side of the two systems of the wire ropes 10A, 20B with a space in the first direction, wherein the main body portion 4 is bendable in the first direction due to a tension state of the two systems of the wire ropes 20A, 20B by bending the operating portion 6 in the first direction.

## Description

### TECHNICAL FIELD

The present invention relates to an elongated medical device such as a catheter device or endoscope.

### BACKGROUND ART

Catheter devices inserted into the heart through blood vessels need to properly guide a distal end portion of a flexible catheter main body to a target site in the heart. Furthermore, endoscopes need to properly guide an imaging portion in the distal end to a target site through a nasal cavity or oral cavity, and in particular, there are elongated medical devices including, on the distal end side, a portion that bends in a desired direction.

In a case where the elongated medical device is a catheter device, as the technique for bending a distal end portion of the catheter main body to properly guide it to the target site, there is a known technique for bending the vicinity of the distal end of the catheter main body by performing the operation to pull a wire attached to the distal end of the catheter main body by a handle portion at hand with the fingertip (see Patent Literature 1). Patent Literature 1 discloses the technique for bending the distal end of the catheter main body in two opposing directions by the operation to rotate, with the fingertip, an operating wire holding handle integrally coupled to a wire stopper disk to which a pair of wires is coupled.

### CITATION LIST

### Patent Literature

Patent Literature 1: Japanese Unexamined Patent Application Publication No. 2010-94484

### SUMMARY OF INVENTION

### TECHNICAL PROBLEM

It often requires a relatively large force to pull the wire so as to bend the distal end of the catheter main body. The bending operation of the catheter main body needs to be performed intermittently during the procedure, and the fingertip needs to successively apply a large force, which results in the issue of a burden on an operator (user).

The present invention has been made in view of the above issue and have an object to provide a technique that makes it possible to easily bend the distal end portion of the main body of the elongated medical device.

### SOLUTION TO PROBLEM

In order to solve such an issue, an elongated medical device according to one aspect includes a bend portion that is bendable and is provided on a distal end side, two systems of operating lines coupled to a distal end portion of the bend portion with a space in a predetermined first direction, and an operating portion that is operable by a user operation and is coupled to a proximal end side of the two systems of the operating lines with a space in the first direction, wherein the bend portion is bendable in the first direction due to a tension state of the two systems of the operating lines by bending the operating portion in the first direction.

In the above-described elongated medical device, the operating portion may include two drive side pulleys that are independently drivable by power from the user and have a common rotation axis, the elongated medical device may further include two pairs, with a space in a second direction intersecting the first direction, of the two systems of the operating lines coupled to the distal end portion of the bend portion with the space in the first direction, and a pulley portion having a rotation plane intersecting the drive side pulley, wherein the two drive side pulleys may be movable in the first direction around a rotation axis of the pulley portion, two systems of operating lines, which are coupled to one end side in the first direction on the distal end side of the bend portion and are aligned in the second direction, and two systems of operating lines, which are coupled to another end side in the first direction and are aligned in the second direction, may be placed on different drive side pulleys of the drive side pulleys via the pulley portion, and when the operating portion is bent to move the two drive side pulleys in the first direction around the rotation axis with respect to the pulley portion, the distal end portion of the bend portion may be bendable in the first direction, and when the operating portion is rotated in the second direction to rotate the two drive side pulleys, the distal end portion of the bend portion may be bendable in the second direction.

In the above-described elongated medical device, the bend portion may be provided with a lumen accommodating each of the systems of the operating lines.

In the above-described elongated medical device, the bend portion may include a lumen through which a predetermined device is inserted or a medicinal solution is injected, and the elongated medical device may include an opening for drawing a tube extending along the lumen outward in a radial direction from the bend portion.

In the above-described elongated medical device, the bend portion may include a lumen through which a predetermined device is inserted or a medicinal solution is injected, and on an extension of a central axis of the bend portion, a fixed shaft serving as a rotation center of the pulley portion may be provided with a first through-hole for inserting a tube extending along the lumen, and the operating portion may be provided with a second through-hole for inserting the tube.

In the above-described elongated medical device, the second through-hole may be formed such that an inner diameter on the proximal end side is larger than an inner diameter on the distal end side.

### ADVANTAGEOUS EFFECTS OF INVENTION

According to the disclosed embodiments, the distal end portion of the main body of the elongated medical device may be easily bent.

### BRIEF DESCRIPTION OF DRAWINGS

FIG. 1 is a perspective view of a catheter device according to an embodiment.
FIG. 2 is a cross-sectional view of the catheter device.
FIG. 3 is a cross-sectional view of a main body portion of the catheter device along an A-A line illustrated in FIG. 1.
FIG. 4 is a front view of a distal end portion of the catheter device.
FIG. 5 is a schematic configuration diagram of the catheter device.
FIG. 6 is a cross-sectional view of an operating portion of the catheter device.
FIG. 7 is a routing diagram of wire ropes of the catheter device.
FIG. 8 is a diagram illustrating upward and downward bending operations of the catheter device.
FIG. 9 is a diagram illustrating a bending operation of the catheter device in a lateral direction.
FIG. 10 is a perspective view of the catheter device according to a modification.
FIG. 11 is a cross-sectional view of the catheter device.
FIG. 12 is a schematic configuration diagram of the catheter device.
FIG. 13 is a cross-sectional view of the operating portion of the catheter device.

### DESCRIPTION OF EMBODIMENTS

An elongated medical device according to an embodiment will be described with reference to the drawings, but the disclosed embodiments are not limited only to the embodiment described in the drawings. Furthermore, in this description, the "distal end side" and "distal end direction" refer to the side and direction in which a portion of the elongated medical device inserted into a body cavity is located. The "proximal end side" and "proximal end direction" refer to the side and direction in which an operating portion is located, which applies power to bend the elongated medical device. Further, the "distal end" refers to an end portion on the distal end side of any member or portion, and the "proximal end" refers to an end portion on the proximal end side of any member or portion.

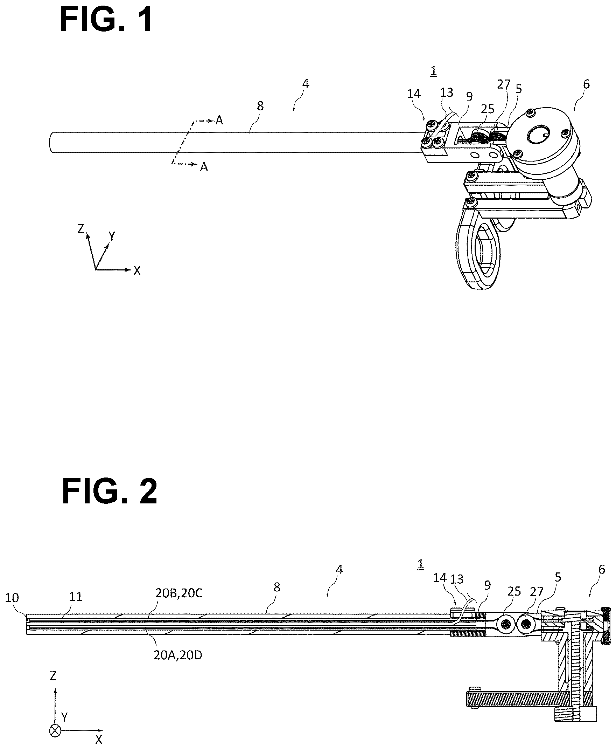

FIG. 1 is a perspective view of a catheter device according to an embodiment. FIG. 2 is a cross-sectional view thereof. Furthermore, an X axis, a Y axis, and a Z axis illustrated in the figures are used as appropriate to describe the directions in the figures. Further, the directions of the X axis, the Y axis, and the Z axis in each figure basically indicate the identical directions. Here, the plus direction of the X axis (the direction of the arrow indicating the X axis) indicates the proximal end side, and the opposite indicates the distal end side. Moreover, in the state (referred to as a reference state) where the longitudinal direction of a catheter device 1 is horizontal, the Z-axis direction is a vertical direction, the X axis is the longitudinal direction, and the Y axis is the transverse direction perpendicular to the longitudinal direction. Therefore, the Z-axis direction may be referred to as the upward and downward direction, and the Y-axis direction may be referred to as the right-and-left direction or the lateral direction.

The catheter device 1 is an example of an elongated medical device and is, for example, a minimally invasive surgical instrument that is guided through a blood vessel to a predetermined site in the heart to give predetermined treatment. The catheter device 1 includes a main body portion 4, a pulley fixing portion 9, a coupling portion 5, and an operating portion 6. A portion (distal end portion) on the distal end side of the main body portion 4 enables upward and downward bending operations and rightward and leftward bending operations.

The main body portion 4 is an example of a bend portion and includes a shaft 8 and a metal ring 10. The shaft 8 may be made of synthetic resin or the like, for example. The metal ring 10 is a ring-shaped metallic member coupled to the distal end of the shaft 8. The pulley fixing portion 9 is coupled to the proximal end side of the shaft 8.

The coupling portion 5 couples the operating portion 6 to the pulley fixing portion 9 so as to enable rotation in upward and downward directions. According to the present embodiment, the operating portion 6 is rotatable in upward and downward directions around a rotation axis of a pulley portion 27. The operating portion 6 is a portion for a user (e.g., surgeon) to operate the distal end side of the main body portion 4 so as to perform upward and downward bending operations and rightward and leftward bending operations.

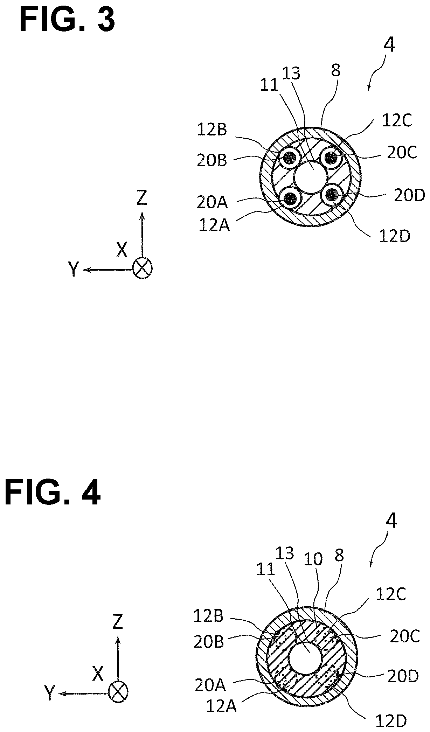

FIG. 3 is a cross-sectional view of the main body portion of the catheter device, representing the cross-section along an A-A line illustrated in FIG. 1. Inside the shaft 8 of the main body portion 4, there are lumens (inner cavities, passages) 12A to 12D for inserting a plurality of (four in this example) wire ropes (examples of operating lines) and a treatment lumen 11 for a device used for treatment on the distal end side of the main body portion 4 or for supplying a medicinal solution to the distal end side. According to the present embodiment, a wire rope 20A is inserted through the lumen 12A, a wire rope 20B is inserted through the lumen 12B, a wire rope 20C is inserted through the lumen 12C, and a wire rope 20D is inserted through the lumen 12D. The lumens 12A to 12D may be made of tubes. Furthermore, according to the present embodiment, the treatment lumen 11 is formed of a tube 13.

FIG. 4 is a front view of the distal end portion of the catheter device. In the distal end portion of the main body portion 4, the metal ring 10 is coupled to the shaft 8. With the metal ring 10, the distal end side of the lumens 12A to 12D is sealed while the distal end side of the treatment lumen 11 is open. The wire ropes 20A to 20D are coupled to the metal ring 10. The wire ropes 20A to 20D may be coupled to the metal ring 10 by a method such as welding or gluing.

According to the present embodiment, the wire ropes 20A, 20D are coupled at the same height position (lower side) in the Z-axis direction in the metal ring 10 when viewed from the front of the distal end portion of the main body portion 4 with the treatment lumen 11 at the center, and the wire ropes 20B, 20C are coupled at the same height position (upper side) in the Z-axis direction. That is, the wire ropes 20A and 20D and the wire ropes 20B and 20C are coupled to the metal ring 10 with a space in the Z-axis direction (an example of a first direction).

The wire ropes 20A, 20B are coupled at the same position (left side) in the Y-axis direction in the metal ring 10, and the wire ropes 20C, 20D are coupled at the same position (right side) in the Y-axis direction. That is, the wire ropes 20A and 20B and the wire ropes 20C and 20D are coupled to the metal ring 10 with a space in the Y-axis direction (an example of a second direction) intersecting the Z axis.

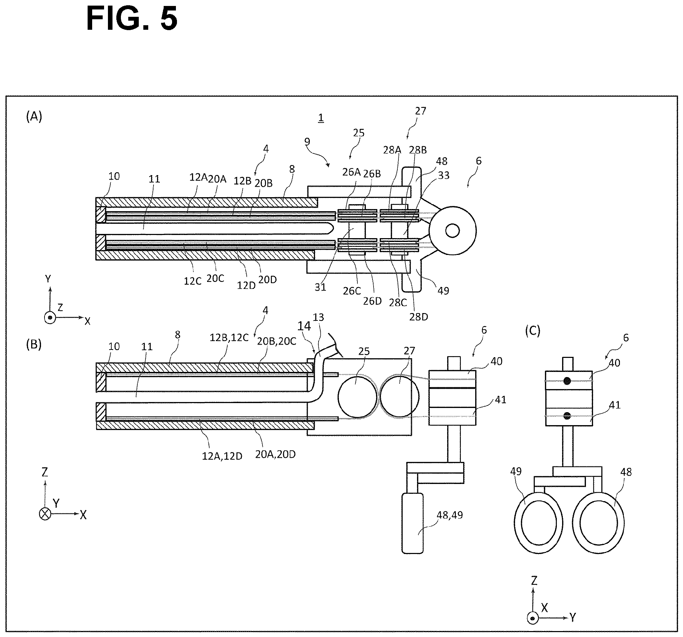

FIG. 5 is a schematic configuration diagram of the catheter device; (A) is a top view thereof, (B) is a side view of the catheter device facing in the Y-axis direction, and (C) is a side view from the X-axis direction.

In the pulley fixing portion 9, a pulley portion 25 and the pulley portion 27 are rotatably supported around rotary shafts 31, 33, respectively. When the catheter device 1 is in the reference state, the rotation axes of the pulley portion 25 and the pulley portion 27 are axes parallel to the Y axis, and the rotation planes are X-Z planes (vertical planes). Furthermore, an upper portion of the pulley fixing portion 9 is provided with an opening 14 for drawing upward the tube 13 communicating with the treatment lumen 11 of the main body portion 4.

The pulley portion 25 includes four pulleys 26A to 26D. The pulleys 26A to 26D are disk-shaped members having groove portions formed on the outer peripheries on which the wire ropes may be placed and are rotatable independently around the rotary shaft 31. According to the present embodiment, the rotary shaft 31 is a fixed shaft that is not rotatable, and each of the pulleys 26A to 26D is rotatable with respect to the rotary shaft 31. The pulleys 26A to 26D may be made of, for example, medical stainless steel.

The pulley portion 27 includes four pulleys 28A to 28D. The pulleys 28A to 28D are disk-shaped members having groove portions formed on the outer peripheries on which the wire ropes may be placed and are rotatable independently around the rotary shaft 33. According to the present embodiment, the rotary shaft 33 is a fixed shaft that is not rotatable, and each of the pulleys 28A to 28D is rotatable with respect to the rotary shaft 33. The pulleys 28A to 28D may be made of, for example, medical stainless steel.

According to the present embodiment, the diameters (pulley diameters) of the pulleys 26A to 26D and 28A to 28D are, for example, 9 mm.

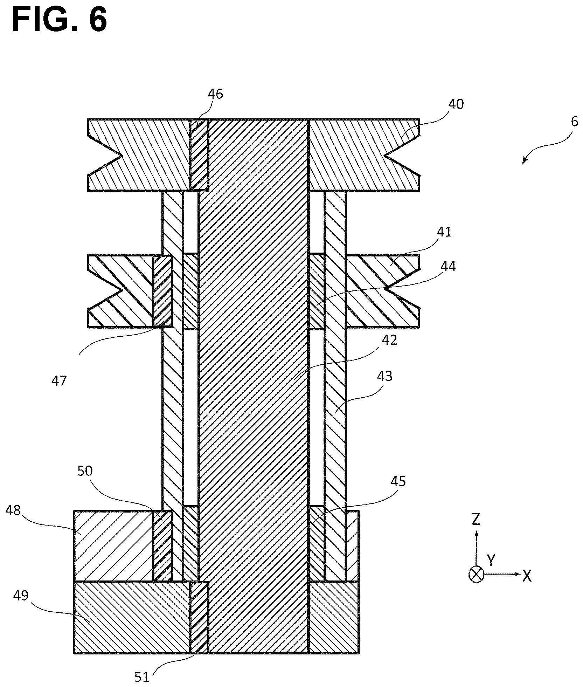

FIG. 6 is a cross-sectional view of the operating portion of the catheter device. The operating portion 6 includes proximal end side pulleys (drive side pulleys) 40, 41, a solid shaft 42 and a hollow shaft 43 as examples of an operating axis, bearings 44, 45, keys 46, 47, 50, 51, and finger hooks 48, 49.

The proximal end side pulleys 40, 41 are disk-shaped members having groove portions formed on the outer peripheries on which the wire ropes may be placed and are each rotatable independently around the operating axis. In the state (the state illustrated in FIG. 5) where the catheter device 1 is in the reference state and the operating portion 6 is not rotated upward or downward, the proximal end side pulley 40 is located above the proximal end side pulley 41 (in the plus direction of the Z axis), the rotation axes thereof are common rotation axis (vertical axis) in parallel with the Z axis, and their rotation planes are an X-Y plane (horizontal plane). Therefore, even when the operating portion 6 is rotated upward or downward with respect to the main body portion 4, the rotation axes of the proximal end side pulleys 40, 41 are perpendicular to the rotary shaft 33 of the pulley portion 27, in other words, the rotation planes of the proximal end side pulleys 40, 41 are perpendicular to the rotation plane of the pulley portion 27. The proximal end side pulley 40 and the proximal end side pulley 41 may be made of, for example, medical stainless steel.

Two systems of the wire ropes 20A, 20D are hooked to the groove portions of the proximal end side pulley 40 and one end of each of the two systems of the wire ropes is fixed thereto. Two systems of the wire ropes 20B, 20C are hooked to the groove portions of the proximal end side pulley 41 and one end of each of the two systems of the wire ropes is fixed thereto. Here, the wire ropes 20A to 20D are formed, for example, by twisting a plurality of wires (strands).

Each of the finger hooks 48, 49 is a portion through which one finger of the hand of the user of the catheter device 1 is inserted to apply the driving force so as to operate the catheter device 1. According to the present embodiment, the finger hooks 48, 49 are configured to be located at a position away from the operating axis on the distal end side in the reference state.

The solid shaft 42 is inserted into a hollow portion of the hollow shaft 43 and is rotatably mounted around the inner periphery of the hollow shaft 43 via the bearings 44, 45. The proximal end side pulley 40 is fixed to the solid shaft 42 via the key 46 and is rotatable together with the solid shaft 42. The proximal end side pulley 41 is fixed to the hollow shaft 43 via the key 47 and is rotatable together with the hollow shaft 43.

The finger hook 48 is fixed to the hollow shaft 43 via the key 50 and is rotatable together with the hollow shaft 43. The finger hook 49 is fixed to the solid shaft 42 via the key 51 and is rotatable together with the solid shaft 42. Therefore, when the user rotates the finger hook 48, the proximal end side pulley 41 rotates in the identical direction, and when the user rotates the finger hook 49, the proximal end side pulley 40 rotates in the identical direction. According to the present embodiment, the finger hooks 48, 49 are located in a position away from the drive shafts (the solid shaft 42, the hollow shaft 43) so that the proximal end side pulleys 40, 41 are rotatable with a relatively small force.

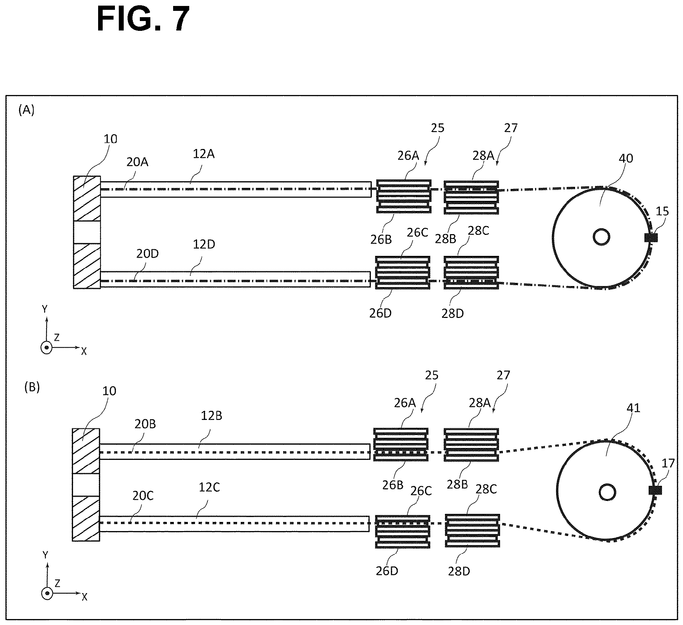

Next, the routing of the wire ropes 20A to 20D, which are passed between the metal ring 10 of the main body portion 4 and the proximal end side pulleys 40, 41 in the catheter device 1, will be described. FIG. 7 is a routing diagram of the wire rope; (A) illustrates the routing of the wire rope between the metal ring 10 and the proximal end side pulley 40, and (B) illustrates the routing of the wire rope between the metal ring 10 and the proximal end side pulley 41.

As illustrated in FIG. 7(A), one end of the wire rope 20A is coupled to the position of the metal ring 10 on the plus side of the Y axis in the distal end of the main body portion 4. The wire rope 20A passes through the lumen 12A from the metal ring 10 and, via the lower groove of the pulley 26A and the upper groove of the pulley 28A, is placed on the proximal end side pulley 40. The other end of the wire rope 20A is fixed to the proximal end side pulley 40 with a fixing member 15.

One end of the wire rope 20D is coupled to the position of the metal ring 10 on the minus side of the Y axis in the distal end of the main body portion 4. The wire rope 20D passes from the metal ring 10 to the proximal end side pulley 40 through the lumen 12D, via the lower groove of the pulley 26D and the upper groove of the pulley 28D. The other end of the wire rope 20D is fixed to the proximal end side pulley 40 with the fixing member 15.

With this configuration, when the finger hook 49 is rotated counterclockwise, the proximal end side pulley 40 rotates counterclockwise, the force is applied to the metal ring 10 coupled via the wire ropes 20A, 20D so as to rotate counterclockwise, and as a result, the distal end of the main body portion 4 is bent to the minus side of the Y axis.

As illustrated in FIG. 7(B), one end of the wire rope 20B is coupled to the position of the metal ring 10 on the plus side of the Y axis in the distal end of the main body portion 4. The wire rope 20B passes from the metal ring 10 to the proximal end side pulley 41 through the lumen 12B and via the upper groove of the pulley 26B and the lower groove of the pulley 28A. The other end of the wire rope 20B is fixed to the proximal end side pulley 41 with a fixing member 17.

One end of the wire rope 20C is coupled to the position of the metal ring 10 on the minus side of the Y axis in the distal end of the main body portion 4. The wire rope 20C passes from the metal ring 10 to the proximal end side pulley 41 through the lumen 12C and via the upper groove of the pulley 26C and the upper groove of the pulley 28C. The other end of the wire rope 20C is fixed to the proximal end side pulley 41 with the fixing member 17.

With this configuration, when the finger hook 48 is rotated counterclockwise, the proximal end side pulley 41 rotates counterclockwise, the force is applied to the metal ring 10 coupled via the wire ropes 20B, 20C so as to rotate counterclockwise, and as a result, the distal end of the main body portion 4 is bent to the minus side of the Y axis.

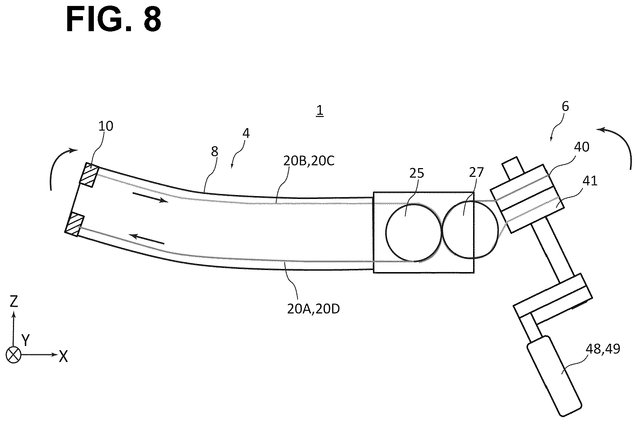

Next, the upward and downward bending operations of the catheter device 1 will be described. FIG. 8 is a diagram illustrating the upward and downward bending operations of the catheter device.

When the operating portion 6 is rotated upward, the operating portion 6 rotates around the rotary shaft 33 of the pulley 27. Accordingly, the two systems of the wire ropes 20B, 20C coupled to the lower proximal end side pulley 41 are pulled to the proximal end side, while the two systems of the wire ropes 20A, 20D coupled to the upper proximal end side pulley 40 are loosened. As a result, the plus side (upper side) of the Z axis of the metal plate 10 coupled to the wire ropes 20B, 20C is pulled to the proximal end side, while the minus side (lower side) of the Z axis of the metal plate 10 coupled to the wire ropes 20A, 20D is easily moved in a direction away from the proximal end side. As a result, the distal end of the main body portion 8 is bent upward. Furthermore, the wire ropes 20A to D are inserted through the lumens 12A to 12D, respectively, and restricted in position so as not to have any adverse effect on the surrounding portions in the shaft 8.

Conversely, when the operating portion 6 is rotated downward, the distal end of the main body portion 8 is bent downward by the four systems of the wire ropes 20A, 20B, 20C, 20D.

According to the present embodiment, the operating portion 6 itself may be rotated to bend the distal end side of the main body portion 4 upward or downward and therefore, as compared with the case where it is bent by operation with the force of the fingertip, a relatively large force may be applied easily, and the distal end side of the main body portion 4 may be easily bent upward or downward.

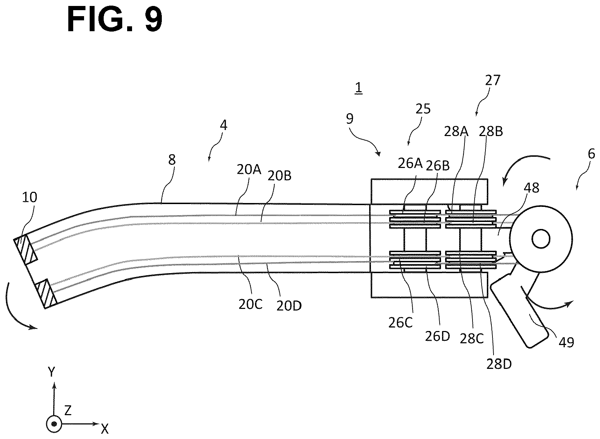

Next, the bending operation of the catheter device 1 in the lateral direction will be described. FIG. 9 is a diagram illustrating the bending operation of the catheter device in the lateral direction.

In the catheter device 1, when the finger hook 48 is rotated counterclockwise, the proximal end side pulley 41 rotates counterclockwise and the force is applied to the metal ring 10 coupled via the wire ropes 20B, 20C so as to bend counterclockwise, and as a result, the distal end portion of the main body portion 4 bends in the minus direction of the Y axis while, when the finger hook 48 is rotated clockwise, the proximal end side pulley 41 rotates clockwise and the force is applied to the metal ring 10 coupled via the wire ropes 20B, 20C so as to bend clockwise, and as a result, the distal end portion of the main body portion 4 bends in the plus direction of the Y axis.

When the finger hook 49 is rotated counterclockwise, the distal end portion of the main body portion 4 bends in the minus direction of the Y axis while, when the finger hook 49 is rotated clockwise, the distal end portion of the main body portion 4 bends in the plus direction of the Y axis.

Furthermore, when the finger hook 48 and the finger hook 49 are moved in the identical direction, the force is applied to rotate the distal end portion of the main body portion 4 in the identical direction so as to achieve the bending operation in the lateral direction. Specifically, when the finger hook 48 and the finger hook 49 are rotated in a counterclockwise direction from the reference position, the distal end portion of the main body portion 4 may bend in the minus direction of the Y axis as illustrated in FIG. 9, and when the finger hook 48 and the finger hook 49 are rotated in a clockwise direction from the reference position, the distal end portion of the main body portion 4 may bend in the plus direction of the Y axis.

As described above, according to the above embodiment, the upward and downward bending operations and the bending operations in the lateral direction of the distal end portion of the main body portion 4 are realized by the four systems of the wire ropes (20A, 20B, 20C, 20D). Furthermore, the combination of the operations on the operating portion 6 for the upward and downward bending operations and the operations on the operating portion 6 for the bending operations in the lateral direction may achieve bending operations combining the upward and downward bending operations and the bending operations in the lateral direction.

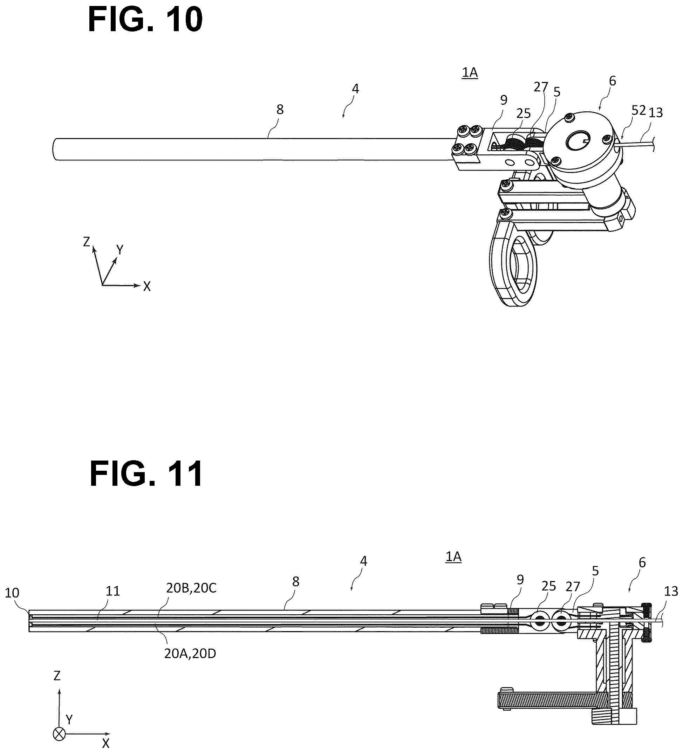

Next, the catheter device according to a modification will be described. FIG. 10 is a perspective view thereof, and FIG. 11 is a cross-sectional view. Furthermore, the same portions as those in the catheter device according to the above embodiment are denoted by the same reference symbols and duplicated descriptions are omitted.

A catheter device 1A according to the modification is a device that allows the tube 13 extending along the treatment lumen 11 to be drawn from the extension of the central axis of the main body portion 4 instead of being drawn through the opening 14 of the pulley fixing portion 9 in the catheter device 1 according to the above-described embodiment.

In the catheter device 1A, the tube 13 may be drawn to the proximal end side through a through-hole 52 formed in the operating portion 6.

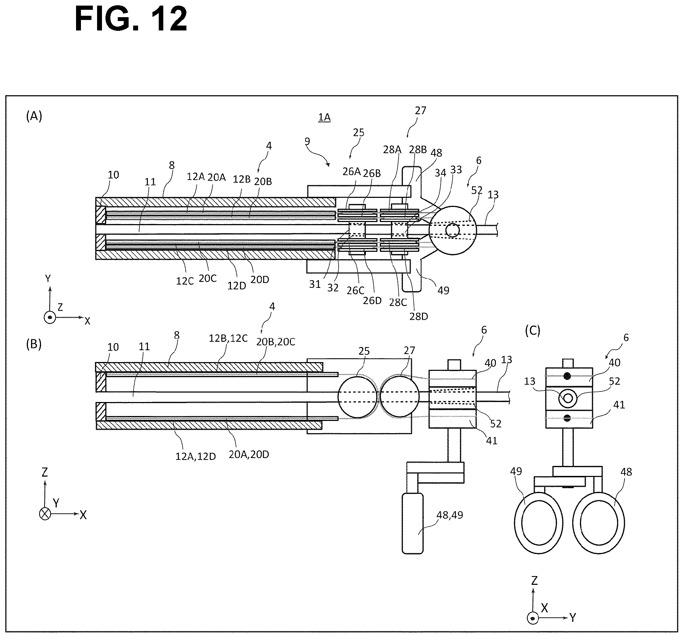

FIG. 12 is a schematic configuration diagram of the catheter device; (A) is a top view thereof, (B) is a side view of the catheter device facing the Y-axis direction, and (C) is a side view from the X-axis direction.

The rotary shaft 31 of the pulley portion 25 is provided with a through-hole 32 (a first through-hole) that allows the tube 13 to extend on the extension of the central axis of the main body portion 4. Furthermore, the rotary shaft 32 of the pulley portion 27 is provided with a through-hole 34 (the first through-hole) that allows the tube 13 to extend on the extension of the central axis of the main body portion 4. Further, the through-hole 52 (a second through-hole) that allows the tube 13 to extend is formed on the extension of the central axis of the main body portion 4 in the operating portion 6.

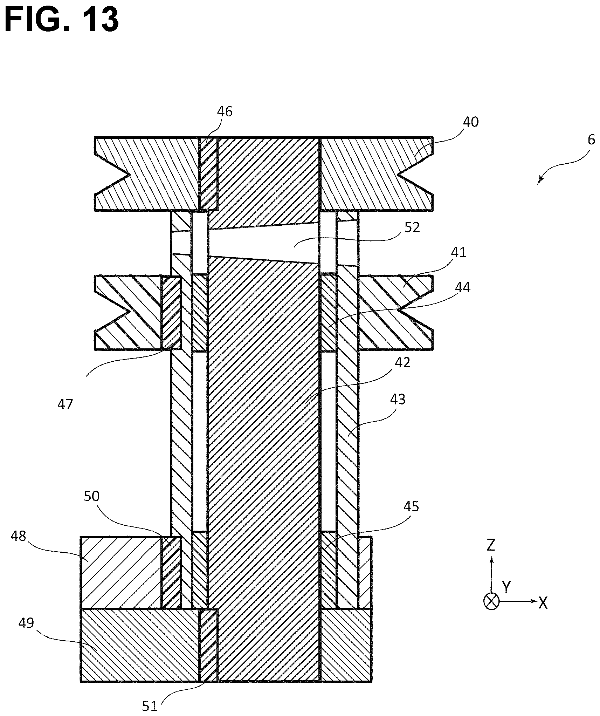

FIG. 13 is a cross-sectional view of the operating portion of the catheter device. The through-hole 52 extending on the extension of the central axis of the main body portion 4 is formed in a drive shaft portion between the proximal end side pulley 40 and the proximal end side pulley 41 in the operating portion 6. The maximum diameter of the through-hole 52 on the proximal end side is larger than the maximum diameter on the distal end side.

Here, the drive shafts (the solid shaft 42, the hollow shaft 43) of the operating portion 6 rotate and move in upward and downward directions in accordance with user operations. In this case, the through-hole 52 also rotates and moves in the upward and downward directions together with the movement of the drive shaft. The movement range of the through-hole 52 is wider on the proximal end side. According to the present embodiment, as described above, the maximum diameter of the through-hole 52 on the proximal end side, where the movement range is wider, is larger than the maximum diameter on the distal end side, and therefore it is possible to reduce the situation where, when the operating portion 6 is rotated or moved, the outer periphery portion of the through-hole 52 comes into contact with the tube 13 and bends the tube 13.

The technique disclosed in this description is not limited to the embodiment and the modification described above, but may be deformed into various forms without departing from the scope thereof, for example, may be modified as described below.

For example, in the example used according to the embodiment or the modification described above, the four systems of the wire ropes 20A, 20B, 20C, 20D are separate, i.e., four wire ropes, but the present invention is not limited thereto and, for example, two wire ropes attached to each of the identical proximal end side pulleys 40 and 41 may be configured as one wire rope.

According to the embodiment or the modification described above, the routing of the wire ropes is obtained such that, when the operating portion 6 is bent upward, the distal end side of the main body portion 4 is bent upward, but the present invention is not limited thereto and, for example, the number of pulley portions may be changed to obtain the routing of the wire ropes such that, when the operating portion 6 is bent upward, the distal end portion of the main body portion 4 is bent downward.

The pulleys of the pulley portions are independently rotatable around the rotary shafts, but the disclosed embodiments are not limited thereto and at least some of the pulleys may be non-rotatable. In this case, the groove portion of the non-rotatable pulley may include a member having a low frictional resistance that allows the wire rope to easily slide.

Although the wire rope is used as an operating line, a wire may be used or an elastic string member may be used instead of the wire rope.

Although the shaft 8 of the main body portion 4 includes one treatment lumen, the present invention is not limited thereto, and a plurality of treatment lumens may be included.

Although the configuration is such that the rotation plane of the proximal end side pulley is perpendicular to that of the pulley portion provided on the rotary shaft that rotates the proximal end side pulley in the upward and downward directions, but the present invention is not limited thereto and the configuration may be such that the rotation planes intersect each other at any angle other than 90 degrees.

Although the catheter device is described as an example of the elongated medical device, the present invention is not limited thereto and, for example, any elongated medical device including a bendable portion in the distal end, such as an endoscope, may be applied.

The present invention is not limited to the configuration according to the embodiment described above, but is indicated by the scope of claims and is intended to include the meaning equivalent to the scope of claims and all the modifications within the scope.

### REFERENCE SIGNS LIST

1, 1A Catheter device
4 Main body portion
8 Shaft
9 Pulley fixing portion
10 Metal ring
11 Treatment lumen
12A to 12D Lumen
13 Tube
14 Opening
15, 17 Fixing member
20A to 20D Wire rope
25 Pulley portion
26A to 26D Pulley
27 Pulley portion
28A to 28D Pulley
31, 33 Rotary shaft
32, 34 Through-hole
40, 41 Proximal end side pulley
42 Solid shaft
43 Hollow shaft
44, 45 Bearing
46, 47, 50, 51 Key
48, 49 Finger hook
52 Through-hole

## Claims

1. An elongated medical device comprising:
a bend portion that is bendable and is provided on a distal end side;
two systems of operating lines coupled to a distal end portion of the bend portion with a space in a predetermined first direction; and
an operating portion that is operable by a user operation and is coupled to a proximal end side of the two systems of the operating lines with a space in the first direction, wherein
the bend portion is bendable in the first direction due to a tension state of the two systems of the operating lines by bending the operating portion in the first direction.

2. The elongated medical device according to claim 1,
wherein the operating portion includes two drive side pulleys that are independently drivable by power from the user and have a common rotation axis,
the elongated medical device further comprising:
two pairs, with a space in a second direction intersecting the first direction, of the two systems of the operating lines coupled to the distal end portion of the bend portion with the space in the first direction; and
a pulley portion having a rotation plane intersecting the drive side pulley, wherein
the two drive side pulleys are movable in the first direction around a rotation axis of the pulley portion,
two systems of operating lines, which are coupled to one end side in the first direction on the distal end side of the bend portion and are aligned in the second direction, and two systems of operating lines, which are coupled to another end side in the first direction and are aligned in the second direction, are passed on different drive side pulleys of the drive side pulleys via the pulley portion, and
when the operating portion is bent to move the two drive side pulleys in the first direction around the rotation axis with respect to the pulley portion, the distal end portion of the bend portion is bendable in the first direction, and when the operating portion is rotated in the second direction to rotate the two drive side pulleys, the distal end portion of the bend portion is bendable in the second direction.

3. The elongated medical device according to claim 2, wherein the bend portion is provided with a lumen accommodating each of the systems of the operating lines.

4. The elongated medical device according to claim 2 or 3,
wherein the bend portion includes a lumen through which a predetermined device is inserted or a medicinal solution is injected,
the elongated medical device comprising an opening for drawing a tube extending along the lumen outward in a radial direction from the bend portion.

5. The elongated medical device according to claim 2 or 3, wherein
the bend portion includes a lumen through which a predetermined device is inserted or a medicinal solution is injected, and
on an extension of a central axis of the bend portion, a fixed shaft serving as a rotation center of the pulley portion is provided with a first through-hole for inserting a tube extending along the lumen, and the operating portion is provided with a second through-hole for inserting the tube.

6. The elongated medical device according to claim 5, wherein the second through-hole is formed such that an inner diameter on the proximal end side is larger than an inner diameter on the distal end side.
